# EUROPEAN PATENT SPECIFICATION

(11) **EP 1 196 160 B1**
(45) Date of publication and mention of the grant of the patent: **21.12.2005**
(21) Application number: 00943001.8
(22) Date of filing: 21.06.2000
(51) Int. Cl.: A61K 31/195, A61P 29/00

(54) **A SYNERGISTIC COMBINATION: GABAPENTIN AND PREGABALIN**
SYNERGISTISCHE KOMBINATION: GABAPENTIN UND PREGABALIN
COMBINAISON SYNERGIQUE DE GABAPENTINE ET DE PREGABALINE

(30) Priority: 02.07.1999 US 142215 P
(43) Date of publication of application: 17.04.2002
(73) Proprietor: Warner-Lambert Company LLC, Morris Plains, NJ 07950 (US)
(72) Inventor: BRUMMEL, Roger, N., Holland, MI 49423 (US); SINGH, Lakhbir, Cambridgeshire, CB7 3SP (GB)
(74) Representative: Tesch, Rudolf
(86) International application number: PCT/US2000/017039
(87) International publication number: WO 2001/001983

(56) References cited:
- WO-A-00/53225
- WO-A-99/08667
- DE-A- 19 802 327

## Description

### BACKGROUND OF THE INVENTION

Compounds of formula 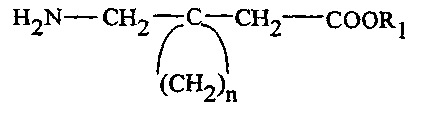 wherein R₁ is hydrogen or a lower alkyl radical and n is 4, 5, or 6 are known in United States Patent Number 4,024,175 and its divisional United States Patent Number 4,087,544. The uses disclosed are: protective effect against cramp induced by thiosemicarbazide; protective action against cardiazole cramp; the cerebral diseases, epilepsy, faintness attacks, hypokinesia, and cranial traumas; and improvement in cerebral functions. The compounds are useful in geriatric patients. The patents are hereby incorporated by reference.

Compounds of formula 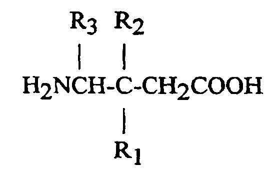 or a pharmaceutically acceptable salt thereof wherein R₁ is a straight or branched alkyl group having from 1 to 6 carbon atoms, phenyl or cycloalkyl having from 3 to 6 carbon atoms; R₂ is hydrogen or methyl; and R₃ is hydrogen, or carboxyl are known in United States Patent Number 5,563,175 and its various divisionals.

### SUMMARY OF THE INVENTION

The instant invention is a pharmaceutical composition of synergistic effect which comprises a therapeutically effective amount of gabapentin or a pharmaceutically acceptable salt or hydrate thereof and therapeutically effective amount of pregabalin or a pharmaceutically acceptable salt or hydrate thereof, with the proviso that said pharmaceutical does not contain a sodium channel blocker, or gabapentin/pregabalin/ opioid, gabapentin/pregabalin/NSAID, gabapentin/pregabalin/naproxen.

The pharmaceutical composition comprises gabapentin in the form of the free acid and pregabalin is in the form of the free acid.

The pharmaceutical composition is gabapentin in a ratio of 1:1000 and pregabalin is from 1:1000.

The pharmaceutical composition with a ratio of gabapentin to pregabalin from 1:1 to 1000:1 1 by weight.

The preferred pharmaceutical composition with a ratio from 1:1 1 to 250:1 by weight.

The invention is the manufacture of a medicament for the treatment of pain in a mammal in need thereof comprising administering a therapeutically effective amount of gabapentin or a pharmaceutically acceptable salt or hydrate thereof and a therapeutically effective amount of pregabalin or a pharmaceutically acceptable salt or hydrate thereof in unit dosage form, with the proviso that said pharmaceutical does not contain a sodium channel blocker, or gabapentin/pregabalin/ opioid, gabapentin/pregabalin/NSAID, gabapentin/pregabalin/naproxen.

It is also the manufacture of a medicament for the treatment of pain in a mammal in need thereof comprising concomitant administration of gabapentin or a pharmaceutically acceptable salt or hydrate thereof and pregabalin or a pharmaceutically acceptable salt or hydrate thereof, with the proviso that said pharmaceutical does not contain a sodium channel blocker, or gabapentin/pregabalin/ opioid, gabapentin/pregabalin/NSAID, gabapentin/pregabalin/naproxen.

The method comprising administering gabapentin in the amount of from 5 to 250 mg and pregabalin in the amount of from 5 to 25 mg.

The range of the types of pain is wide including chronic and acute types.

### BRIEF DESCRIPTION OF THE INVENTION

Figures 1 and 2 show the effect of a fixed dose 1:1 1 ratio of gabapentin and pregabalin on the maintenance of CITH.
Figures 3 and 4 show the effect of a fixed dose 10:1 ratio of gabapentin and pregabalin on the maintenance of CITH.

### DETAILED DESCRIPTION OF THE INVENTION

Gabapentin is the generic name for the marketed product Neurontin®. The chemical name is 1-(aminomethyl)-cyclohexaneacetic acid. The chemical structure of the compound is:

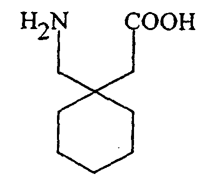

Pregabalin is the generic name for (S)-3-(aminomethyl)-5-methylhexanoic acid. The chemical structure of the compound is: 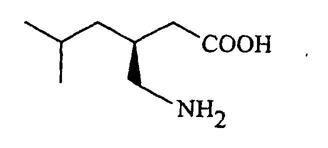 It is also known as CI-1008 and as S-(+)-3-IBG.

Formerly, it was thought that gabapentin and pregabalin were the same in all pain models as one antagonist blocked both; therefore, a similar result was expected.

However, surprising differences have now been observed in an inflammatory model of pain.

The present invention relates to pharmaceutical compositions. These compositions have a synergistic effect in the treatment of pain. Advantages of these compositions include fewer side effects as lower dosages are needed. This increases patient compliance with the beneficial result of better control of pain.

The drugs can be administered together in the same dosage unit or can be prepared in separate dosage units administered at the same time. Different forms of dosage units can be used, i.e., a tablet of gabapentin and an injection of pregabalin.

One particular advantage of the instant invention is the fact that no cross tolerance between the two compounds has been observed.

The synergistic composition of this invention utilizes any GABA analogs. A GABA analog is a compound derived from or based upon gamma-aminobutyric acid.

### METHODS FOR COMBINATION STUDIES IN THE CARRAGEENAN-INDUCED THERMAL HYPERALGESIA MODEL

### Animals

Male Sprague-Dawley rats (175-200 g), obtained from Bantin and Kingman (Hull, U.K.), were housed in groups of 6 under a 12-hour light/dark cycle (lights on at 07 h 00 min) with food and water ad libitum. All experiments were carried out by an observer unaware of drug treatments.

### Evaluation of Thermal Hyperalgesia

Thermal hyperalgesia was assessed using the rat plantar test (Ugo Basile, Italy) following a modified method of Hargreaves K., Dubner R., Brown F., Flores C., and Joris J., A new and sensitive method for measuring thermal nociception in cutaneous hyperalgesia, *Pain* 1988;32:77-88. Male Sprague-Dawley rats (70-90 g) were habituated to the apparatus which consisted of three individual perspex boxes on an elevated glass table. A mobile radiant heat source located under the table was focused onto the desired paw and withdrawal latencies (PWL) recorded. PWLs were taken 3 times for both hind paws of each animal, the mean of which represented baselines for right and left hind paws. At least 5 minutes were allowed between each PWL for an animal. The apparatus was calibrated to give a PWL of approximately 10 seconds. There was an automatic cut-off point of 20 seconds to prevent tissue damage. After baseline PWLs were determined, animals received an intraplantar injection of carrageenan (100 µL of 20 mg/mL) into the right hind paw. PWL were reassessed following the same protocol as above 2 hours postcarrageenan (this time point represented the start of peak hyperalgesia) to ascertain that hyperalgesia had developed. Test compounds were then administered as combinations at 2.5-hour post-carrageenan and PWL taken again at 1, 2, and 4-hour postdrug.

### Combination Studies

Dose responses to gabapentin and pregabalin were first performed alone in the carrageenan-induced thermal hyperalgesia (CITH) model. The dose response data for both compounds were used to determine theoretical additive lines using the method described by Berenbaum M.C., What is synergy? *Pharmacological Reviews* 1989;41:93-141. Combinations of gabapentin and pregabalin were determined following a fixed ratio design, where the doses of both compounds vary in fixed dose ratios of 1:1 and 10:1. A dose response to the combination was performed following this design and compared to the theoretical additive line.

### Drugs

Gabapentin and pregabalin were synthesised at Parke-Davis (Ann Arbor, USA). λ-Carrageenan were obtained from Sigma (Poole, UK). All compounds were dissolved in water except carrageenan which was dissolved in isotonic saline. Gabapentin and pregabalin combinations were administered in the same solution. Drug administrations were made in a volume of 1 mL/kg.

### Data Analysis

Data for dose responses were subjected to a one-way analysis of variance (ANOVA) followed by a Dunnett's t-test. The dose response data for both compounds were used to determine theoretical additive lines as described by Berenbaum 1989.

The figures show the synergy between gabapentin and pregabalin by comparing the theoretical addition and the synergetic responses.

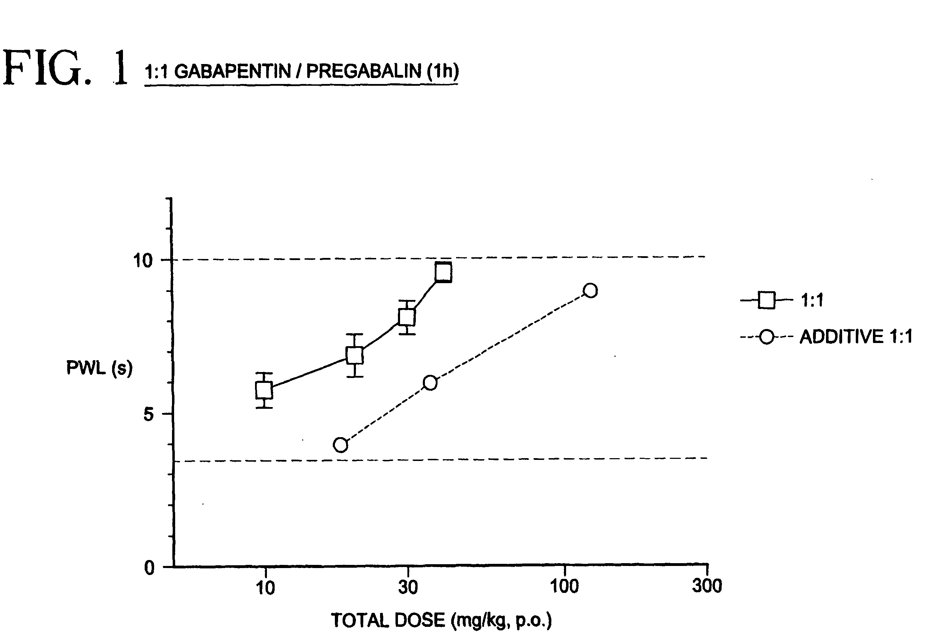

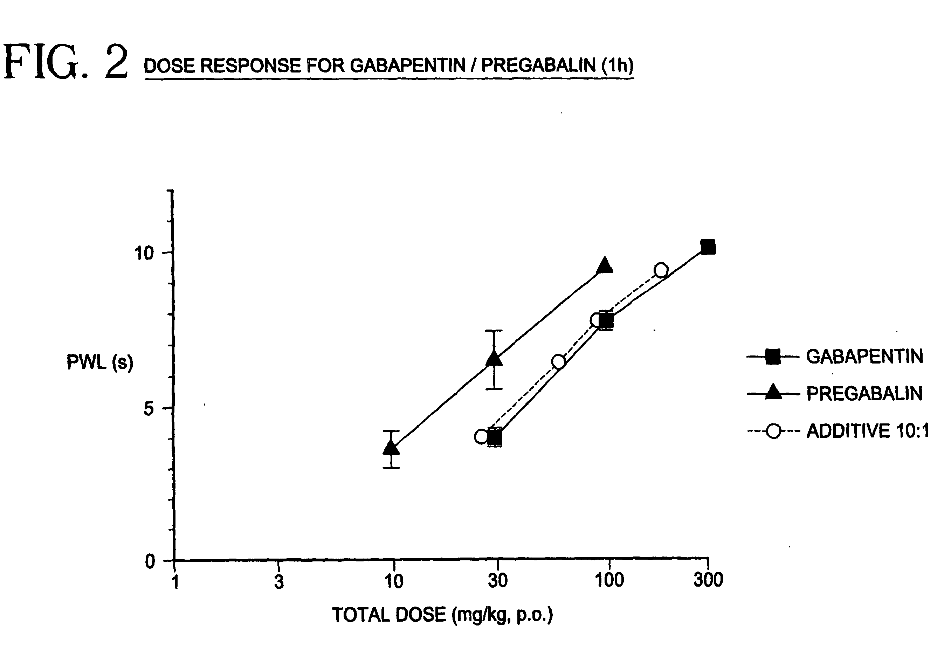

Figures 1 and 2. Effect of a 1:1 Fixed Dose Ratio of Gabapentin: Pregabalin on the Maintenance of Carrageenan-Induced Thermal Hyperalgesia. Dose response data for gabapentin and pregabalin alone (a). Fixed dose ratio of 1:1 gabapentin:pregabalin combination (b). The theoretical additive line was calculated from the dose response data in (a). All compounds were administered P.O. and PWL to plantar test were examined 1-hour post drug administration. Results are expressed as mean PWL(s) (vertical bars represent ± SEM).

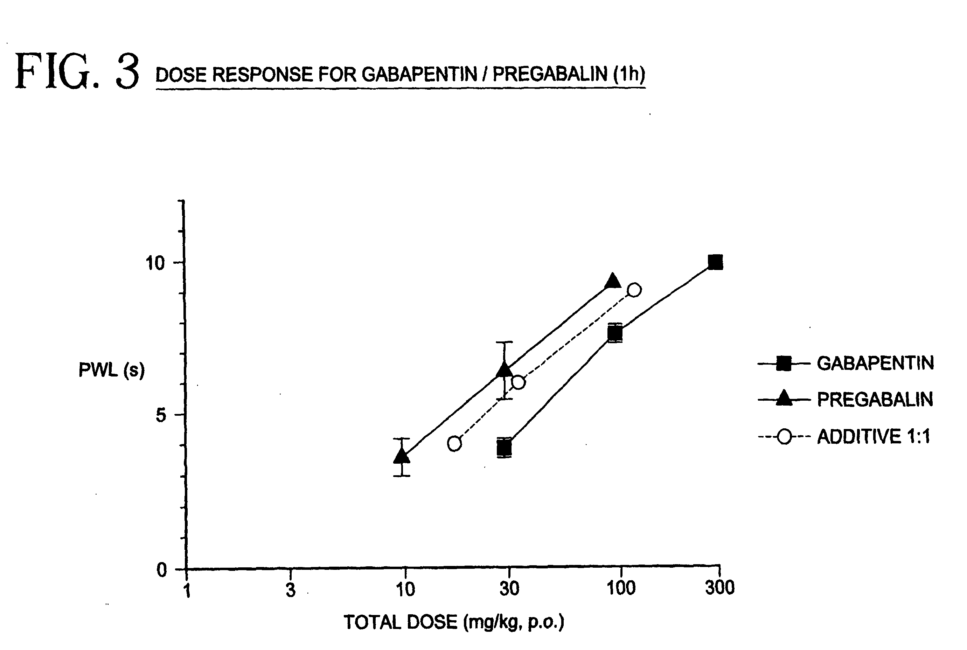

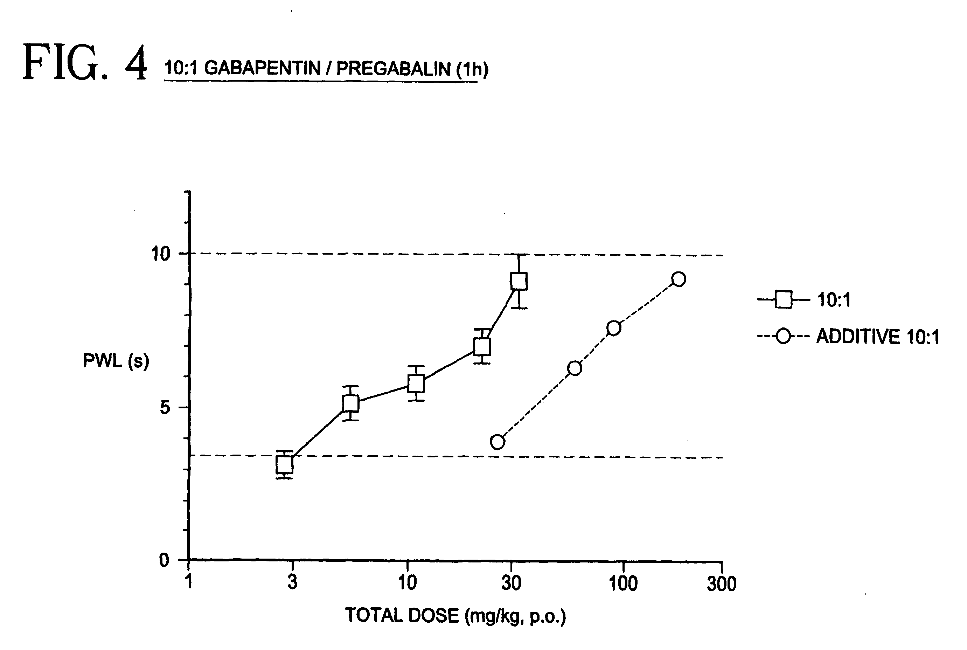

Figures 3 and 4. Effect of a 10:1 Fixed Dose Ratio of Gabapentin: Pregabalin on the Maintenance of Carrageenan-Induced Thermal Hyperalgesia. Dose response data for gabapentin and pregabalin alone (a). Fixed dose ratio of 10:1 gabapentin:pregabalin combination (b). Theoretical additive line was calculated from the dose response data in (a). All compounds were administered P.O. and PWL to plantar test were examined 1-hour post-drug administration. Results are expressed a mean PWL(s) (vertical bars represent ± SEM).

The instant invention is useful in a range of types of pain. It refers to acute as well as chronic pain.

Acute pain is usually short-lived (e.g. postoperative pain). Chronic pain is usually defined as pain persisting from 3 to 6 months and includes somatogenic pains and psychogenic pains. Other types of pain are caused by injury or infection of peripheral sensory nerves. It includes, but is not limited to pain from peripheral nerve trauma, herpes virus infection, diabetes mellitus, causalgia, plexus avulsion, neuroma, limb amputation, and vasculitis. Neuropathic pain is also caused by nerve damage from chronic alcoholism, human immunodeficiency virus infection, hypothyroidism, uremia, or vitamin deficiencies.

Psychogenic pain is that which occurs without an organic origin such as low back pain, atypical facial pain, and chronic headache.

Other types of pain are: inflammatory pain, osteoarthritic pain, trigeminal neuralgia, cancer pain, diabetic neuropathy, restless leg syndrome, acute herpetic and postherpetic neuralgia, causalgia, brachial plexus avulsion, occipital neuralgia, gout, phantom limb, bum, and other forms of neuralgia, neuropathic and idiopathic pain syndrome.

A skilled physician will be able to determine the appropriate situation in which subjects will find the synergistic combination useful.

The compounds of the present invention can be prepared and administered in a wide variety of oral and parenteral dosage forms. Thus, the compounds of the present invention can be administered by injection, that is, intravenously, intramuscularly, intracutaneously, subcutaneously, intraduodenally, or intraperitoneally. Also, the compounds of the present invention can be administered by inhalation, for example, intranasally. Additionally, the compounds of the present invention can be administered transdermally. It will be obvious to those skilled in the art that the following dosage forms may comprise as the active component, either a compound of Formula I or a corresponding pharmaceutically acceptable salt of a compound of Formula I.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component.

In tablets, the active component is mixed with the carrier having the necessary binding properties in suitable proportions and compacted in the shape and size desired.

The powders and tablets preferably contain from five or ten to about seventy percent of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogenous mixture is then poured into convenient sized molds, allowed to cool, and thereby to solidify.

Liquid form preparations include solutions, suspensions, and emulsions, for example, water or water propylene glycol solutions. For parenteral injection liquid preparations can be formulated in solution in aqueous polyethylene glycol solution.

Aqueous solutions suitable for oral use can be prepared by dissolving the active component in water and adding suitable colorants, flavors, stabilizing and thickening agents as desired.

Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsules, tablet, cachet, or lozenge itself, or it can be the appropriate number of any of these in packaged form.

The quantity of active component in a unit dose preparation may be varied within wide limits. For practical purposes, it is present in a concentration of about 10% in a solid composition and about 2% in a primary liquid composition. In medical use the drug may be administered 1 to 3 times daily as, for example, as capsules. The composition can, if desired, also contain other compatible therapeutic agents.

In therapeutic use, the compounds utilized in the pharmaceutical method of this invention are administered at the initial dosage of about 1 mg to about 1000 mg/kg daily. A daily dose range of about 1 mg to about 500 mg/kg is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day, if desired.

The relative amounts of the active ingredients in the combination may vary within a wide range.

The synergistic combination may contain a ratio of about 1:1 to about 1000:1; preferably 1:1 to 500:1 and particularly from 1:1 to 250:1 parts by weight of gabapentin or a pharmaceutically acceptable salt or hydrate thereof to pregabalin or a pharmaceutically acceptable salt or hydrate thereof.

The synergistic compositions of the instant invention are prepared by methods known in the pharmaceutical industry. For example, the compositions may be prepared by admixing the active ingredient with inert, non-toxic carriers or diluents (e.g. cellulose, silicic acid, stearine, polyomyspyrsolidone, talc, starch, etc.). The compositions may also contain well known additives (e.g. emulsifying or suspending agents, dyes, salts for controlling the osmotic pressure, buffers, etc.)

The following examples are for illustrative purposes and are not intended to limit the scope of the invention.

### EXAMPLES

### Capsules

50 mg, 100 mg, 125 mg, 200 mg, 250 mg, 300 mg, or 400 mg
Gabapentin, 125 mg
Pregabalin, 50 mg
Lactose USP, Anhydrous q.s. or 250 g
Sterotex Powder HM, 5 g

Combine the compound and the lactose in a tumble blend for 2 minutes, blend for 1 minute with the intensifier bar, and then tumble blend again for 1 minute. A portion of the blend is then mixed with the Sterotex powder, passed though a #30 screen and added back to the remainder of the blend. The mixed ingredients are then blended for 1 minute, blended with the intensifier bar for 30 seconds, and tumble blended for an additional minute. The appropriately sized capsules are filled with 141 mg, 352.5 mg, or 705 mg of the blend, respectively, for the 50 mg, 125 mg and 250 mg containing capsules.

### Tablets

5 mg, 100 mg, 200 mg, 300 mg, 400 mg, 500 mg, or 600 mg
Gabapentin, 200 mg
Pregabalin, 5 mg
Corn Starch NF, 200 g
Cellulose, Microcrystalline, 46 g
Sterotex Powder HM, 4 g
Purified Water q.s. or 300 mL

Combine the com starch, the cellulose, and the compound together in a planetary mixer and mix for 2 minutes. Add the water to this combination and mix for one minute. The resulting mix is spread on trays and dried in a hot air oven at 50°C until a moisture level of 1 to 2 percent is obtained. The dried mix is then milled.

### Injectables

Gabapentin, 125 mg
Pregabalin, 5 mg
Water for Injection USP, q.s.

The compound or a suitable salt thereof is dissolved in water and passed through a 0.2-micron filter. Aliquots of the filtered solution are added to ampoules or vials, sealed and sterilized.

### Capsules

100 mg, 300 mg, 400 mg
Gabapentin, 95 mg
Pregabalin, 5 mg
Lactose
Corn Starch
Talc
Gelatin
Titanium Dioxide

### Capsules

Gabapentin or Pregabalin 100 mg, 300 mg, 400 mg, 600 mg
Gabapentin: 100 mg and inactive ingredients gelatin and titanium dioxide
   300 mg and inactive ingredients gelatin, titanium dioxide, and yellow iron oxide
   400 mg and inactive ingredients gelatin, red iron oxide, titanium
   dioxide, and yellow iron oxide
The above amounts can be adjusted as needed.

## Claims

1. A pharmaceutical composition of synergistic effect which comprises a therapeutically effective amount of gabapentin or a pharmaceutically acceptable salt or hydrate thereof and therapeutically effective amount of pregabalin or a pharmaceutically acceptable salt or hydrate thereof
with a proviso that said pharmaceutical composition does not contain a sodium channel blocker, or gabapentin/pregabalin/opioid, gabapentin/pregabalin/NSAID, gabapentin/pregabalin/naproxen.

2. A pharmaceutical composition according to Claim 1 which comprises gabapentin in the form of the free acid and pregabalin is in the form of the free acid.

3. A pharmaceutical composition according to Claim 1 wherein gabapentin is in a ratio from 1:1000 and pregabalin is from 1:1000.

4. A pharmaceutical composition according to claim 1 with a ratio of gabapentin to pregabalin from 1:1 to 1000:1 by weight.

5. A pharmaceutical composition according to claim 1 with a ratio from 1:1 to 250:1 by weight.

6. Use of a therapeutically effective amount of gabapentin or a pharmaceutically acceptable salt or hydrate thereof and a therapeutically effective amount of pregabalin or a pharmaceutically acceptable salt or hydrate thereof for the manufacturing of pharmateuticals for the treatment of pain
with the proviso that pharmaceuticals does not contain a sodium channel blocker, or gabapentin/pregabalin/opioid, gabapentin/pregabalin/NSAID, gabapentin/pregabalin/naproxen.

7. Use of a therapeutically effective amount of gabapentin or a pharmaceutically acceptable salt or hydrate thereof and a therapeutically effective amount of pregabalin or a pharmaceutically acceptable salt or hydrate thereof for the manufacturing of pharmateuticals for concomitant administration of said compounds for the treatment of pain
with the proviso that pharmaceuticals does not contain a sodium channel blocker, or gabapentin/pregabalin/opioid, gabapentin/pregabalin/NSAID, gabapentin/pregabalin/naproxen.

8. Use according to claim 6 or 7 of gabapentin in the amount of from 5 to 250 mg and pregabalin in the amount of from 5 to 25 mg.

9. Use according to claim 6 or 7 wherein the pain is selected from hyperalgesia, allodynia, and inflammatory.

## Patentansprüche

1. Pharmazeutische Zusammensetzung mit synergistischer Wirkung, umfassend eine therapeutisch wirksame Menge Gabapentin oder dessen pharmazeutisch akzeptablen Salzes oder Hydrats und eine therapeutisch wirksame Menge Pregabalin oder dessen pharmazeutisch akzeptablen Salzes oder Hydrats, unter der Bedingung, dass die pharmazeutische Zusammensetzung keinen Natriumkanalblocker, Gabapentin/Pregabalin/Opioid, Gabapentin/Fregabalin/NSAID oder Gabapentin/Pregabalin/Naproxen enthält.

2. Pharmazeutische Zusammensetzung nach Anspruch 1, umfassend Gabapentin in Form der freien Säure und Pregabalin in Form der freien Säure.

3. Pharmazeutische Zusammensetzung nach Anspruch 1, worin Gabapentin in einem Verhältnis von 1:1000 und Pregabalin in einem Verhältnis von 1:1000 vorhanden ist.

4. Pharmazeutische Zusammensetzung nach Anspruch 1, wobei das Gewichtsverhältnis von Gabapentin zu Pregabalin von 1:1 bis 1000:1 beträgt.

5. Pharmazeutische Zusammensetzung nach Anspruch 1, wobei das Gewichtsverhältnis von 1:1 bis 250:1 beträgt.

6. Verwendungeiner therapeutisch wirksamen Menge Gabapentin oder dessen pharmazeutisch akzeptablen Salzes oder Hydrats, und einer therapeutisch wirksamen Menge Pregabalin oder dessen pharmazeutisch akzeptablen Salzes oder Hydrats zur Herstellung von Arzneimitteln zur Behandlung von Schmerzen, unter der Bedingung, dass die Arzneimittel keinen Natriumkanalblocker, Gabapentin/Progabalin/Opioid, Gabapentin/Pregabatin/NSAID oder Gabapentin/Pregabalin/Naproxen enthalten.

7. Verwendung einer therapeutisch wirksamen Menge Gabapentin oder dessen pharmazeutisch akzeptablen Salzes oder Hydrats, und einer therapeutisch wirksamen Menge Pregabalin oder dessen pharmazeutisch akzeptablen Salzes oder Hydrats zur Herstellung von Arzneimitteln zur begleitenden Verabreichung der genannten Verbindungen zur Behandlung von Schmerzen, unter der Bedingung, dass die Ameimittel keinen Natriumkanalblocker, Gabapentin/Pregabalin/Opioid, Gabapentin/Pregabalin/NSAID oder Gabapentin/Pregabalin/Naproxen enthalten.

8. Verwendung nach Anspruch 6 oder 7, wobei Gabapentin in einer Menge von 5 bis 250 mg und Pregabalin in einer Menge von 5 bis 25 mg verwendet wird.

9. Verwendung nach Anspruch 6 oder 7, wobei die Schmerzen ausgewählt sind aus Hyperalgesie, Allodynie und Entzündungsschmerzen.

## Revendications

1. Composition pharmaceutique d'effet synergique comprenant une quantité thérapeutiquement efficace de gabapentine ou un sel ou un hydrate pharmaceutiquement acceptable de celle-ci et une quantité thérapeutiquement efficace de prégabaline ou un sel ou un hydrate pharmaceutiquement acceptable de celle-ci, à condition que ladite composition pharmaceutique ne contienne pas d'inhibiteur des canaux sodiques, ni de gabapentine/pregabaline/opioïde, nl de gabapentine/prégabaline/AINS, ni de gabapentine/ prégabaline/naproxène.

2. Composition pharmaceutique selon la revendication 1, qui comprend de la gabapentine sous forme d'acide libre et de la prégabaline sous forme d'acide libre.

3. Composition pharmaceutique selon la revendication 1 dans laquelle la gabapentine est présente dans un rapport 1: 1000 et la prégabaline est présente dans un rapport 1: 1000.

4. Composition pharmaceutique présentant un rapport entre la gabapentine et la prégabaline compris entre 1 : 1 et 1000 : 1 en poids.

5. Composition pharmaceutique selon la revendication 1 présentant un rapport compris entre 1 ; 1 et 250 : 1 en poids.

6. Utilisation d'une quantité thérapeutiquement efficace de gabapentine ou un sel ou un hydrate pharmaceutiquement acceptable de celle-ci et d'une quantité thérapeutiquement efficace de prégabaline ou un sel ou un hydrate pharmaceutiquement acceptable de celle-ci pour la fabrication de produits pharmaceutiques destinés au traitement de la douleur, à condition que les produits pharmaceutiques ne contiennent pas d'inhibiteur des canaux sodiques ni de gabapentine/prégabaline/opioïde, ni de gabapentine/prégabaline/AINS, ni de gabapentine/prégabaline/naproxène.

7. Utilisation d'une quantité thérapeutiquement efficace de gabapentine ou un sel ou un hydrate pharmaceutiquement acceptable de celle-ci et d'une quantité thérapeutiquement efficace de prégabaline ou un sel ou un hydrate pharmaceutiquement acceptable de celle-ci pour la fabrication de produits pharmaceutiques en vue d'une administration concomitante desdits composés pour le traitement de la douleur, à condition que les produits pharmaceutiques ne contiennent pas d'inhibiteur des canaux sodiques ni de gabapentine/prégabaline/opioïde, ni de gabapentine/ prégabaline/AINS, ni de gabapentine/prégabaline/naproxène.

8. Utilisation selon la revendication 5 ou 7 de gabapentine dans une quantité comprise entre 5 et 250 mg et de prégabaline dans une quantité comprise entre 5 et 25 mg

9. Utilisation selon la revendication 6 ou 7 dans laquelle la douleur est sélectionnée parmi l'hyperalgésie, l'allodynie et la douleur inflammatoire.
